# EUROPEAN PATENT APPLICATION

(11) **EP 4 725 455 A1**
(43) Date of publication of application: **15.04.2026**
(21) Application number: 24383123.7
(22) Date of filing: 14.10.2024
(51) Int. Cl.: A61F 2/28, A61F 2/30, B33Y 80/00, G09B 23/30, A61B 34/10

(54) **COMPUTER IMPLEMENTED METHODS AND SYSTEMS FOR GENERATING MODELS OF BONES, AND FOR ADDITIVE MANUFACTURING OF BONES**

(71) Applicant: Fundació Privada Elisava Escola Universitária, 08002 Barcelona (ES); Universitat Autònoma de Barcelona, 08193 Cerdanyola del Vallès (Barcelona) (ES); Fundació Universitaria Balmes (Universitat de Vic Universitat Central de Catalunya), 08500 Vic (ES); Fundació Institut de Recerca i Innovació en Ciències de la Vida i la Salut a la Catalunya Central, 08500 Vic (ES)
(72) Inventor: Otero Viñas, Marta, Vic (ES); Guri Rios, Júlia, Vic (ES); Crespo Santiago, Juan, Barcelona (ES); Gesualdo, Marco, Barcelona (ES); Carbonell i Mas, Nim, Barcelona (ES); Estrada Fernández, Gerard, Barcelona (ES); Daura Piqué, Pau, Barcelona (ES); de Pouplana Mira, Maria Jesús, Barcelona (ES); Jordana Comín, Xavier, Bellaterra, Cerdanyola del Vallès (Barcelona) (ES); Galtés Vicente, Ignasi, Bellaterra, Cerdanyola del Vallès (Barcelona) (ES); Fradera Pales, Mario, Barcelona (ES); Wückert Loza, Alan, Barcelona (ES); Grothe i Riera, David, Barcelona (ES); Olivella Dalmau, Àlex, Barcelona (ES); Ribot Viayna, Joan, Barcelona (ES)
(74) Representative: Bardehle Pagenberg S.L.

(57) **Abstract**

The present disclosure relates to computer-implemented methods (100) for generating a virtual model of a first section of bone tissue including cortical bone tissue. The method comprises (104) obtaining microstructural cortical parameters including an indication of a number of osteons (210) and of a cross-sectional area of the osteons, a number of lamellae (212) forming the osteons, an indication of locations of osteons in the section of bone tissue and of dominance of the osteons. The method further comprises generating a virtual model using a generative design algorithm comprising (1061) defining a geometric space, (1062) generating a cortical cloud of points, (1064) segmenting the geometric space into substantially circular regions, defining an outer surface of the osteons. The method further comprises (1065) forming consecutive substantially concentric surfaces corresponding to the lamellae until the obtained virtual model satisfies boundary conditions formed by the microstructural cortical parameters.

## Description

### FIELD

The present disclosure relates to computer-implemented methods for generating models of bone, in particular sections of bones or complete bones of mammals, and more particularly of humans. In particular, the present disclosure relates to computer implemented methods for generating input files for a 3D printer and to methods for additive manufacturing of bones.

### BACKGROUND

Research and training in human bone biology face significant challenges due to the limitations in the supply of human biological bone tissue. However, recent developments in additive manufacturing have provided the possibility of obtaining artificial bones which try to imitate biological bones.

Additive manufacturing (AM) or three-dimensional printing, is a process that creates three dimensional objects by depositing materials, usually in layers. Additive manufacturing can shorten the design-manufacturing cycle and thus reduce the production cost and increase the competitiveness. One of the key advantages of 3D printing is the ability to produce very complex shapes or geometries.

3D printing techniques are based on computer-aided design (CAD) and computer-aided manufacturing (CAM), which allow the design and manufacture of synthetic bones resembling biological bones to a certain extent.

However, obtaining artificial bones that actually provide an accurate representation of a biological bone is a challenge, as bone tissue is made of complex structures interacting with each other and which vary for each individual.

The present disclosure is related to the development of new methods and systems which can provide a synthetic or artificial bone which more accurately imitates the behaviour of a real bone, and in particular the behaviour of bones in the case of fracture. The obtained bones may be used for research purposes or may potentially be used as prosthesis.

### SUMMARY

In an aspect of the present disclosure, a computer implemented method is provided for generating a virtual model of a first section of bone tissue including cortical bone tissue. The method comprises obtaining a plurality of macrostructural parameters of the bone tissue and obtaining microstructural cortical parameters of the cortical bone tissue, wherein the microstructural cortical parameters of the cortical bone tissue include an indication of a number of osteons, an indication of a cross-sectional area of the osteons, a number of lamellae forming the osteons, an indication of locations of osteons in the section of bone tissue, and an indication of dominance of the osteons. The method further comprises determining the virtual model of the first section of bone tissue using a generative design algorithm.

The generative design algorithm comprises defining a geometric space of the section of bone tissue, wherein an outer geometric shape of the virtual model is substantially in accordance with the macrostructural parameters. The generative design algorithm further comprises generating a cortical cloud of points, wherein a number of points of the cortical cloud of points corresponds to the number of osteons and wherein a location of the points is derived from the indication of locations of the osteons, and segmenting the geometric space into a plurality of substantially circular regions, wherein each of the regions comprises one of the generated points.

An outer surface of the osteons is based on the substantially circular regions, wherein at an intersection between two adjacent circular regions, the outer surface of the osteons is determined by the substantially circular regions and the level of dominance of the osteons such that a portion of the outer perimeter of a more dominant osteon is defined by the substantially circular region and a portion of the outer perimeter of a less dominant osteon is defined by the substantially circular region of the adjacent osteon.

The generative algorithm further comprises forming of substantially concentric surfaces corresponding to the lamellae within each of the outer perimeters of the osteons until the obtained virtual model satisfies boundary conditions formed by the microstructural cortical parameters.

According to this aspect, the generated virtual model may imitate the microarchitecture of a biological bone. The method uses an input which allows imitation of the fundamental units of cortical bone i.e. osteons. Further, the generated virtual section of the bone tissue imitates not only the disposition of the structural units of the cortical bone, but also the sheets of lamellar bone tissue that form these structural units. Accordingly, the method of the present disclosure is accurate enough to reproduce at a micro scale level the structure of cortical bone. Moreover, by following a generative design based on principles of natural selection of one osteon prevailing over another one, a more realistic representation of the bone may be obtained.

The generated virtual model of bone tissue may be used to generate an input file for a 3D printer and an artificial bone section comprising the microarchitecture of biological bone and which imitates the mechanical properties of biological bone may be obtained.

Further, the method allows to parametrize the microarchitecture of the bones depending on individual conditions of a subject, taking into account the biomechanical aspects of bone.

Macrostructural and microstructural bone features are unique to each individual. At the same time, different individuals sharing similar biological conditions can share similar macro and microstructural features of their bones. The method uses both micro and macrostructural parameters of bone tissue to generate complex structures of long bones which, when printed, comprise the same mechanical properties as a biological bone. As a result, an accurate representation of long bone from microstructure to macrostructure is achieved.

Throughout the present disclosure, an algorithm may be regarded as a series or finite sequence of commands or instructions that a computer or any sort of data processing system must follow.

Throughout the present disclosure, microstructural parameters may refer to parameters of the structure of the bone which may only be observed under a high degree of magnification e.g. using lens or other optical instruments. Macrostructural parameters on the other hand relate describe the large-scale properties and behavior of a bone and which are visible and measurable without advanced microscopes.

Throughout the present disclosure, generating a virtual model of a section of bone tissue may be regarded as generating a digital version of a section of a bone e.g. a long bone of a patient. The digital version may be visualized on a display and may be manipulated through a suitable user interface. The virtual model may include a graphical mathematical coordinate-based representation of an object (in this case, a section of a bone tissue of a patient) in three dimensions via specialized software by manipulating edges, vertices, and polygons in a simulated 3D space. 3D models represent a physical body using a collection of points in 3D space, connected by various geometric entities such as triangles, lines, curved surfaces, etc. Their surfaces may be further defined with texture mapping.

In specific examples of the present disclosure, non-uniform rational basis spline (NURBS) may be used to define the virtual model of (a section) of bone.

In some examples, the plurality of macrostructural parameters and/or the plurality of microstructural parameters obtained as input may be average values for subjects belonging to a population. In some examples, the population of subjects may be based on sex, age and absence or presence of pathological conditions of a subject. In some examples, the pathological parameters may include osteoporosis or arthrosis. That is, in order to obtain a bone structure that mimics human bone according to specific parameters such as age, sex, etc. average parameters for a population may be used.

In some examples, the section of bone tissue may further comprise trabecular bone tissue and the method may comprise obtaining a plurality of microstructural parameters of the trabecular bone tissue, and determining the virtual model of the section of bone tissue comprises generating a virtual model of the trabecular bone tissue.

In further examples, the microstructural trabecular parameters may include indications of a number of trabeculae, an indication of a distribution of trabeculae and an indication of an area and/or cross-section of the trabeculae. By parametrizing the number of trabeculae, their number and distribution, porosity of the bone may be varied as desired as well. E.g. it is known that porosity of trabecular bone is not constant for a given bone but actually varies from the cortical bone inwards, particularly at the transition of cortical bone tissue to trabecular bone tissue. Furthermore, natural trabecular bone tissue grown according to specific load directions and the tissue density is higher along those directions experiencing higher loads.

In some examples, the plurality of macrostructural parameters and/or the plurality of microstructural parameters and/or the plurality of microstructural trabecular parameters may be obtained from user input. That is, a user may use a keyboard, touchscreen, or other interface to provide the input for the generative design algorithm.

In other examples, the input may be obtained from previously generated medical data, particularly medical images. Medical images may include X-ray images, MRI images, high-resolution CT images or others. Data processing may be used to derive the microstructural and/or macrostructural parameters for the bone to be modeled, e.g. the number of osteons, their location, the number of lamellae etc.

In some examples, the obtained parameters may be customizable. A user may customize one or more values of the parameters after a section of a bone tissue has been generated i.e. a user may modify one or more values of the generated section of the long bone.

In some examples, the microstructural cortical parameters may further include indications of thickness of individual lamellae forming the osteons and/or a distance between the individual lamellae forming the osteons. The distance between the individual lamellae may be also referred to as a thickness of interlaminar spaces of the osteons.

In another aspect of the present disclosure, a computer program is provided which comprises instructions which, when the program is executed by a computer, cause the computer to carry out any of the methods disclosed herein. In yet a further aspect, a computer readable medium having the computer program stored thereon is provided. In a further aspect, a carrier signal carrying the computer program is provided.

In yet a further aspect, a computer system is provided which comprises a memory and processor, wherein the processor is configured to carry out the method according to any of the examples disclosed herein.

Additional objects, advantages and features of embodiments of the present disclosure will become apparent to those skilled in the art upon examination of the description, or may be learned by practice.

### BRIEF DESCRIPTION OF THE DRAWINGS

Figure 1 shows a flowchart of a method for generating a virtual model of a first section of bone tissue including cortical bone tissue according to an example of the present disclosure;
Figure 2 schematically illustrates an example of a cross section of a long bone;
Figure 3 schematically illustrates a perspective view of a section of a long bone; and
Figure 4 schematically illustrates an example of a longitudinal cross-section of a long bone;
Figure 5 shows a flowchart showing the steps of a generative design algorithm used for determining the virtual model of the first section of cortical bone tissue according to an example of the present disclosure;
Figures 6a - 6d schematically show steps according to an example of generating a virtual model of a plurality of cortical sections of bone tissue.
Figures 7a and 7bschematically show a perspective view of a generated virtual model of a first section of bone tissue according to an example of the present disclosure;
Figures 8a - 8d schematically show steps of generating a virtual model of a section of trabecular bone tissue using a generative algorithm according to an example of the present disclosure;
Figure 9 schematically shows a top view of a virtual model of a cross section of bone tissue including cortical bone tissue according to another example of the present disclosure;
Figure 10a - 10d schematically show a perspective view of a generated virtual model comprising four sections of cortical bone tissue according to another example of the present disclosure;
Figure 11 shows a flowchart of a method for manufacturing artificial bone tissue; and
Figure 12 schematically represents a synthetic long bone manufactured by additive manufacturing.

### DETAILED DESCRIPTION OF EXAMPLES

Reference will now be made in detail to embodiments of the disclosure, one or more examples of which are illustrated in the drawings. Each example is provided by way of explanation, not as a limitation. In fact, it will be apparent to those skilled in the art that various modifications and variations can be made in the present disclosure without departing from the scope or spirit of the teaching. For instance, features illustrated or described as part of one embodiment can be used with another embodiment to yield a still further embodiment. Thus, it is intended that the present disclosure covers such modifications and variations as come within the scope of the appended claims and their equivalents.

The present disclosure provides a computer implemented method 100 for generating a virtual model of a first section of bone tissue including cortical bone tissue.

The bone tissue including cortical bone tissue may belong to a long bone. In some examples, the long bone may be one of a femur, tibia, fibula, metatarsal, phalange, humerus, radius, ulna or metacarpal.

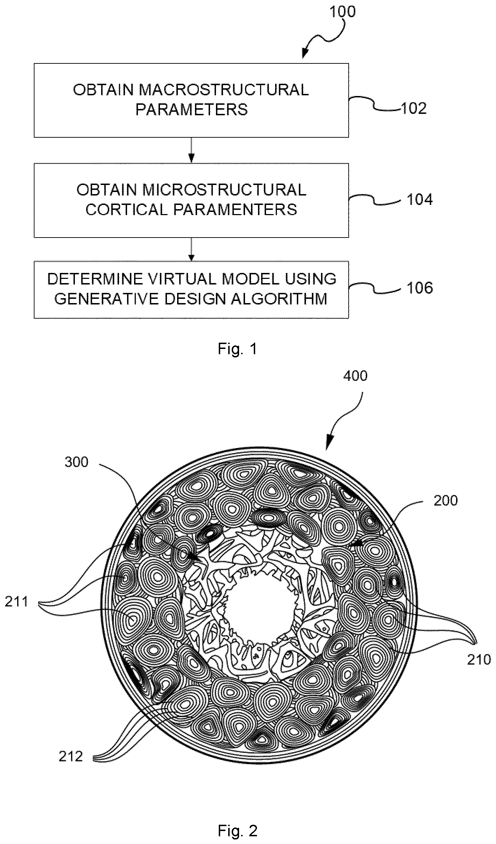

Figure 1 illustrates a flowchart of the method 100 for generating a virtual model of a first section of bone tissue. The method 100 comprises, at block 102, obtaining a plurality of macrostructural parameters of the bone tissue and, at block 104, obtaining microstructural cortical parameters of the cortical bone tissue. It will be clear that the order of the steps may be interchanged freely.

Obtaining a plurality of macrostructural and microstructural parameters may comprise obtaining information regarding the parameters from a data storage. The inputs may be received by a computer system, either wirelessly or through a cable, and the computer system may have a suitable software to generate at least a virtual section of a bone tissue with the received input. Software capable of performing the suitable calculations is known in the art.

The macrostructural and microstructural parameters may be average values for subjects belonging to a population. The one or more values of the parameters may fall within a predetermined range, the range being representative of particular conditions of a subject. Different subjects sharing the same conditions (e.g. osteoporosis) may have different macrostructural and microstructural parameters, however these parameters may be within the same range. In addition, different bone structures at micro level may have the same morphological characteristics at the macro level.

The values of the plurality of macrostructural and microstructural parameters of bone tissue may vary depending on the profile of the bone i.e. the value of the parameters may vary depending on the profile of the subject to whom the bone belongs.

In some examples, the population of subjects may be based on sex, age and absence or presence of pathological conditions of a subject. In some examples, the pathological conditions may include one of osteoporosis or arthrosis. E.g. a long bone may have different microstructural parameters depending on whether the bone belongs to a male or a female, a child or an adult, a healthy person or a person suffering from osteoporosis or arthrosis, etc. The virtual model may be generated using highly specified input values defining bone microstructure.

In some examples, the one or more values of the plurality of microstructural parameters may be obtained from a library of data e.g. data obtained from scientific research or clinical studies.

In some examples, the plurality of parameters may be obtained from user input. A user may, through a user interface, indicate one or more characteristics defining a condition of a particular bone profile e.g. sex, age and health condition, and a personalized input may be obtained on the macrostructural and microstructural parameters of bone tissue, the input comprising one or more values that statistically fall within specified conditions or needs e.g. mean, or median values for the parameters. In some examples, the values may be assigned randomly. The interface may be or may include a tactile display, a computer keyboard, a computer mouse, or other.

In other examples, the plurality of macrostructural and/or microstructural parameters may be derived from one or more medical images. In some examples, the images may be histological images. In some examples, the images may be obtained from micro-computed tomography (CT) or magnetic resonance (MR). In some cases, the parameters may be derived from a plurality of images representative of a particular part of the population. In such a case, mean or median values may be used for the parameters used to define the model of the bone.

The one or more values of the plurality of microstructural parameters may be obtained from an image of a section of a long bone of a subject showing the microstructure of cortical bone area and trabecular bone area. A highly personalized section of a long bone may be obtained, as the input may define the microstructural parameters of the cortical bone of a specific subject i.e. the microstructural parameters may be imitated. The generated section, if printed with the appropriate materials, may be used as grafts, bone substitutes or case studies.

In some examples, obtaining a plurality of macrostructural parameters 102 of bone tissue may comprise obtaining a diameter and/or a perimeter of the long bone. The length and/or the shape of the long bone may also be defined.

Figure 2 schematically shows an example of a cross section of a long bone 400. The cross section shown in figure 2 is perpendicular to the longitudinal direction of the bone. As schematically represented in figure 2, the long bone 400 comprises a cortical or compact region 200 and a trabecular or cancellous region 300.

The cortical region 200 comprises a plurality of osteons 210, also known as Haversian systems. Each of the osteons 210 comprises a Haversian canal 211 defined by an inside of the innermost concentric lamella 212. The lamellae 212 in reality are sheets of mineralized collagen and osteons from healthy cortical bone 200 may be generally formed by 4-20 lamellae 212. However, the number of lamellae may vary depending on multiple factors apart from health conditions of the subject e.g. age, sex or type of bone. The outer boundary of the lamellae is called the cement line, and it delineates each of the osteons from interstitial lamellar bone. An important aspect of the present disclosure to model individual lamellae with surfaces with spaces in between them.

As schematically shown in figure 2, two or more of the osteons 210 of a same cortical region 200 may be formed with different numbers of lamellae 212. In addition, a shape and thickness of the lamellae 212 may also be different between osteons 210 of a same cortical region of the bone.

At block 104 of method 100 of figure 1, the plurality of microstructural cortical parameters i.e. the microstructural parameters of cortical bone, may define osteon microstructure. The osteons are the functional units of cortical bone and simulating their microstructure may provide an input file for a 3D printer which when printed may result in a more realistic bone model i.e. a bone model which shares biomechanical aspects of a real bone.

In particular, in the computer implemented method 100 of the present disclosure, obtaining microstructural cortical parameters of the cortical bone tissue 200 at least includes obtaining an indication of a number of osteons 210, a cross-sectional area of the osteons, a number of lamellae 212 forming the osteons, an indication of locations of osteons in the section of bone tissue, and an indication of dominance of the osteons.

For each of these parameters, the parameters may be defined by specific values, i.e. n osteons, cross-sectional area of X mm², or specific coordinates for their locations. The parameters may however also be defined by approximate values, or ranges i.e. n₁ - n₂ osteons, average cross-sectional area of X mm², and a standard deviation of Y, or cross-sectional area from X - Y mm², whereas positions of the osteons may be defined by indications of densities of osteons in different areas of the model, or may be (more) randomly distributed.

In the method, the obtained input of microstructural parameters takes into account multiple specific conditions of the various microstructural parameters, such that the input on the parameters of the cortical bone 200 provides a comprehensive description of the microarchitecture of the compact region of the bone. The input on the plurality of microstructural parameters of cortical bone may capture the features which make every cortical region unique, therefore allowing obtaining a virtual bone model which accurately mimics biomechanical properties of biological long bones of different constitutions.

Defining a more realistic microstructure of osteons results in the obtention of a model which defines the behavior of each of the osteons more realistically e.g. under mechanical stress. Therefore the behavior of the cortical region under mechanical stress may be more realistically defined as well.

As previously mentioned, the microstructural parameters of cortical bone depend on the profile of the bone i.e. on the profile of a subject. The number of osteons 210 and the cross-sectional area of the osteons 210 forming the cortical bone 200 section may oscillate within a range which is representative of a particular type of subject e.g. age, sex or health conditions.

The one or more values of the obtained microstructural cortical parameters also define the number of lamellae 212 forming each of the osteons 210 of the virtual model. The microstructural cortical parameters also include the location of the osteons within the section of the bone tissue and an indication of dominance of the osteons. The position and distance between each of the osteons 210 within the cortical bone 200 may differ. The position between osteons may be regarded as the distance between the central osteonic canals or Haversian canals 211 with neighboring osteonic canals.

The values of the plurality of microstructural parameters of the cortical bone 200 may be linked to each other, such that variation of one of them may influence one or more of the others. In some examples, the number of osteons 210, the area of each of the osteons and the number of lamellae 212 forming each of the osteons may be linked to each other, such that input on one of the parameters predefines other parameters, or predefines boundary conditions for other parameters.

Further, the microstructural cortical parameters may further include indications of a thickness of individual lamellae 212 forming the osteons 210 and/or a distance between the individual lamellae forming the osteons, i.e. a thickness of the interlamellar spaces of the osteons. E.g. in healthy bone tissues, the lamellae 212 may have a thickness of 1 - 20 µm. The input may define a range of thickness for each of the lamellae, or specific values of thickness of each of the lamella. The input may also include a distribution or function that describes the distribution of lamellae, e.g. the outer lamellae is thicker e.g., twice as thick as other lamellae.

In some examples, the microstructural cortical parameters may further include indications of an area and/or shape of the Haversian canals 211. The Haversian canals 211 may comprise a shape resembling regular or irregular cylinders, each of the cylinders having a different length and volume.

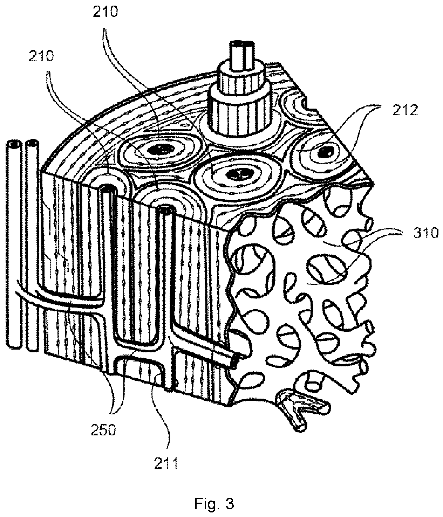

Figure 3 shows a perspective view of an example of a longitudinal cut of a long bone section. The figure schematically shows the cortical 200 and the trabecular region 300. The longitudinal cut shows two adjacent osteons 210 wherein each of the osteons comprises a Haversian canal 211, and wherein the Haversian canals 211 are interconnected via a Volkmann's canal 250. In cortical bone 200, Haversian canals 211 may be interconnected with an adjacent Haversian canal with Volkmann's canals 250.

In some examples, the microstructural cortical parameters of block 104 may also include indications of a number of Volkmann canals 250 connecting the Haversian canals 211. In some examples, the values of the parameters may further define a position of each of the Volkmann's canals 250 e.g. the longitudinal position of the canals in each of the osteons 210.

In further examples, the microstructural cortical parameters may further define a length of the osteons 210 and/or an inclination angle of each of the osteons 210. The concentric surfaces representing the lamellae of the osteons may correspond to cylindrical surfaces having a length of the individual osteons. It will be understood that the terms "cylinder" and "cylindrical" within the scope of the present disclosure do not necessary refer to cylinders having a circular cross-section. Rather

In some examples, the user may define a specific value of a microstructural parameter of the cortical bone 200 e.g. a thickness of the cement line such that it is higher than the rest of the lamellae 212.

Figures 2 and 3 also show a trabecular region 300 or a cancellous region of the long bone. The trabecular region 300 of the bone section is located at the inner part and is surrounded by the cortical region 200.

The trabecular region 300 is formed of trabeculae 310, which consist of groups of parallel lamellae. The plurality of trabeculae 310 forming the trabecular region 300 are connected to each other by nodes. When one side of a trabeculae 300, also referred to as strut, is not joined to a node it is referred to as a termini. The ratio between nodes and termini in a section is an index of spatial connectivity, defining non-uniform porous structures.

Different trabeculae 310 within a same trabecular region have different thicknesses and shapes. The diversity of inputs within a same microstructural parameter allows not only to simulate specific bone conditions, but also to modify them into more personalized models e.g. a wide variety of bones, from an adult osteoporotic bone, to an osteoporotic or healthy bone pertaining to a child may be modeled.

In some examples, the virtual model of a first section of bone tissue may comprise trabecular bone tissue. In these examples, the computer implemented method 100 may further comprise obtaining a plurality of microstructural parameters of the trabecular bone tissue. Determining the virtual model of the section of bone tissue may comprise generating a virtual model comprising the trabecular bone tissue.

In some examples, the microstructural trabecular parameters of bone tissue may include indications of a number of trabeculae, an indication of a distribution of the trabeculae, and an indication of an area and/or cross-section of the trabeculae. As indicated elsewhere, the input may relate to specific values, ranges of values or distribution of values.

The parameters indicating the trabeculae and their distribution indirectly also define porosity of the bone tissue. The indications may define that an area of the pores varies gradually along a transversal direction of the section of the long bone. In some examples, the indications may define a variation in spatial connectivity index along a transversal direction of the long bone. The number of trabeculae and the area of the pores may be a function of the load axes of the bone.

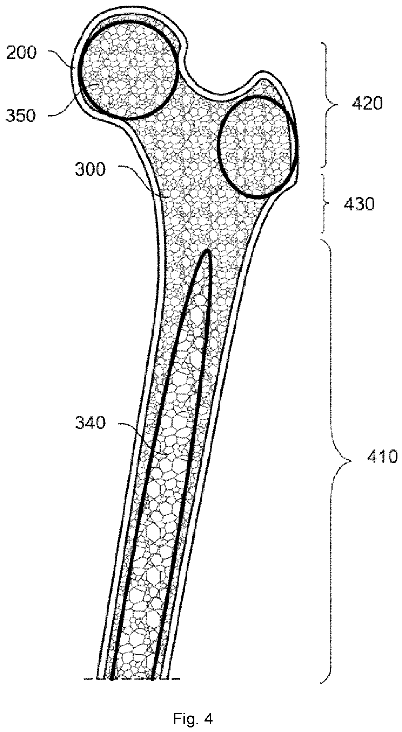

Figure 4 schematically shows a portion of a long bone. The long bone comprises the diaphysis 410, which is the central section of the bone, the proximal and distal epiphysis 420, which are the ends of the long bone, and the metaphasis 430, which is the section of the bone located between the diaphysis 410 and the epiphysis 420.

The long bone comprises a trabecular region 300 and a cortical region 200. Figure 4 schematically shows regions of the trabecular bone with higher density 350 and regions of the trabecular bone with lower density 340.

The density of the trabecular bone regions may vary depending on the mechanical loads that they may endure. As may be seen in the figure, a higher density of trabecular bone tissue may be observed within the head portions of the bone, whereas a lower density of the trabecular tissue is observed in the diaphysis.

In some examples, density of trabecular bone tissue may be defined by the load axes of the bone and also on their position with respect to cortical bone tissue. As mentioned before, the microstructural trabecular parameters may include an indication of a distribution of trabeculae and area or cross-section. The distribution and density of the trabeculae may be derived from the load axes. I.e. user input may include a definition of load axes or axes of increased or decreased density. The algorithm may then distribute the cloud of points of trabeculae in accordance with such axes.

In some examples, the method may further comprise obtaining an input on a plurality of parameters which include indications defining a transitional region between the cortical region and the trabecular region. In these examples, the generated virtual model may include the transitional region.

Biological bone comprises gradient features from cortical to trabecular regions. The focus on microstructural parameters defining the transition region between cortical and trabecular bone is key on the final biomechanics of the artificial bone, and providing a method which takes this into account may allow to generate a section of a long bone, or an entire long bone, which mimics the biomechanical properties of biological bone.

A more realistic behavior of artificial bones may be obtained when representing the gradual transition of the cortical region into the trabecular region. Obtaining such inputs therefore allows generating a virtual section of a long bone which imitates biomechanical behavior thanks to the specificity of its microarchitecture. A synthetic bone which meets highly individualized patient conditions may be manufactured.

After obtaining a plurality of macrostructural 102 and microstructural parameters 104 of bone tissue, the method 100 for generating a virtual model of bone tissue comprises, at block 106, determining the virtual model of the first section of bone tissue using a generative design algorithm. The virtual model may be a digital version of a section of a long bone of a patient. The virtual model of the bone section may be a three-dimensional model.

The section of the long bone may be displayed on a suitable display, and may be digitally or "virtually" manipulated through a suitable electronic user interface e.g. a tactile display, a computer keyboard, a computer mouse, or other.

In some examples, a 3D computer-aided design (CAD) modelling software may be used e.g. Rhino^{™}. In specific examples, the generative design algorithm may be programmed using Grasshopper plug-in for Rhino^{™}.

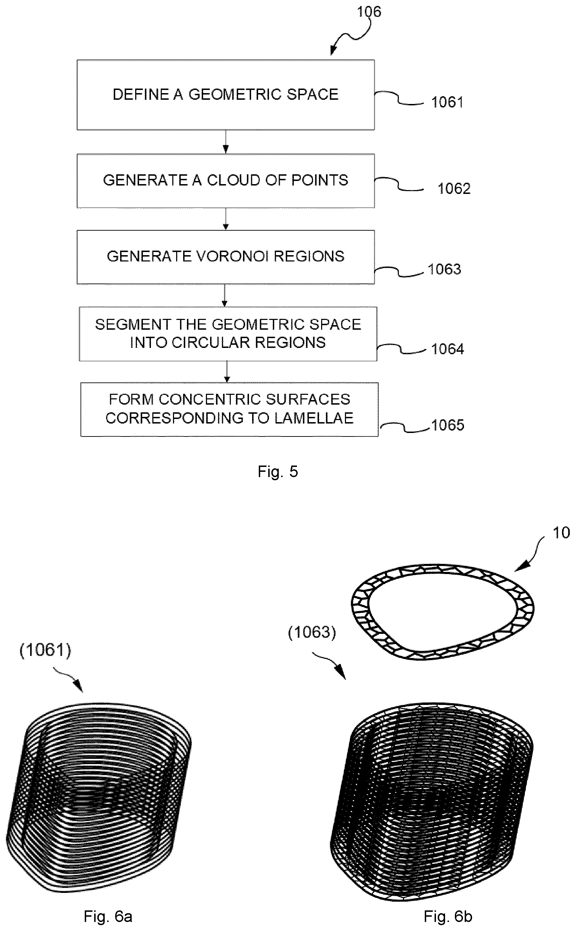

Figure 5 schematically illustrates a flowchart showing the steps of the generative design algorithm used for determining the virtual model 106 of the first section of bone tissue in an example. Figures 6a - 6d schematically show steps according to an example of generating a virtual model of a plurality of cortical sections 10 of bone tissue.

Determining the virtual model of the section of the bone tissue comprises, at step 1061, defining a geometric space of the bone tissue e.g. an area or a volume of the cortical region of the bone tissue. The outer geometric shape of the virtual model is substantially in accordance with the macrostructural parameters.

Figure 6a shows a plurality of geometric spaces defining a plurality of sections of cortical bone tissue. The geometric spaces are substantially ring shaped. A model may comprise more sections space on top of each other.

In examples, the virtual model may also comprise trabecular bone tissue and a geometric space of the trabecular region of the bone tissue may also be generated. The outer perimeter of the trabecular region is then defined by an inner perimeter of the cortical region.

The generative design algorithm comprises, at step 1062, generating a cloud of points, wherein a number of points corresponds to the number of osteons, and a location of points is derived from the locations of the osteons.

In some examples, a Voronoi diagram may be formed from the cloud of points generated in step 1062. In these examples, the method may comprise segmenting the geometric space into Voronoi regions 1063. An example of the generated Voronoi diagram is shown in figure 6b. The edges of the Voronoi regions may be located such that a distance between neighboring points is equidistant.

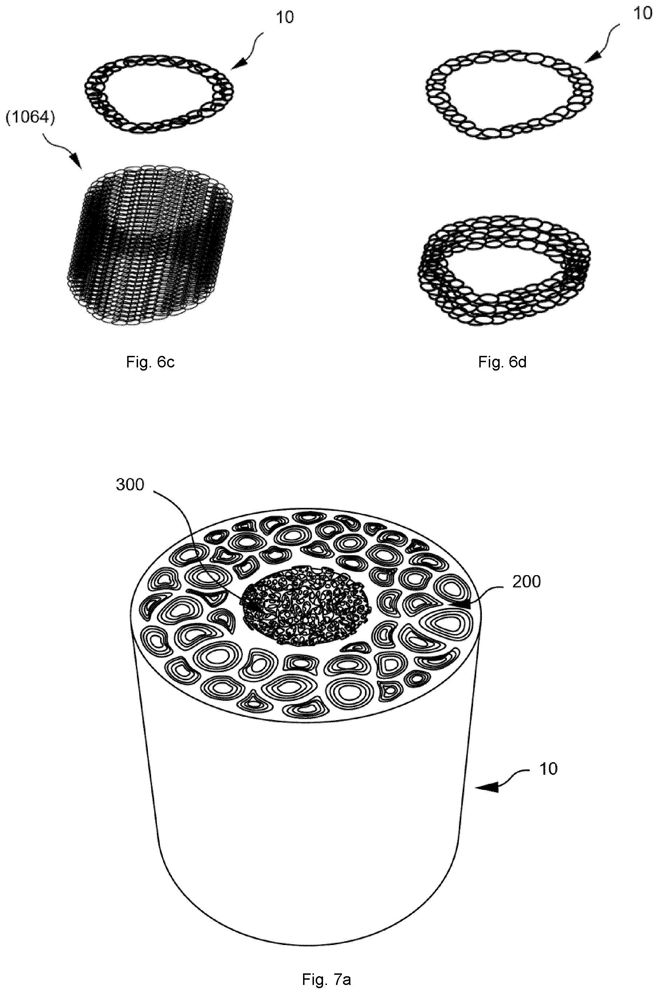

At step 1064, the method comprises segmenting the geometric space into a plurality of substantially circular regions, wherein each of the regions comprises one of the generated points and corresponds to a rounding interpolation of the Voronoi regions. It may be seen in figure 6c, that the substantially circular regions overlap with each other.

Figure 6d schematically shows different osteons comprising different shapes. At a border between two adjacent regions, a shape of the osteons is determined by the level of dominance of the individual osteons. The osteons may have different levels of dominance. When an osteon with a higher level of dominance is adjacent to an osteon with a lower level of dominance, the osteon with lower level of dominance adapts its shape to adapt to the space that to the available space i.e. the space that is not occupied by the osteon with a higher level of dominance. In other words, the outer perimeter of a more dominant osteon is defined by the substantially circular region obtained in the step of figure 6c. The outer perimeter of a less dominant osteon is defined by the substantially circular region of the adjacent osteon (and will thus generally not be circular, but rather obtain a less regular shape).

In some examples, the level of dominance may be randomly assigned to the different points of the cloud of points. In some examples, a plurality of the points may comprise a lower level of dominance i.e. may be defined as primary osteons and the other plurality of points may comprise a higher level of dominance i.e. the osteons defined by those points may be defined as secondary osteons. An accurate representation of biological bone tissue, taking into account the shape of the cortical bone due to the formation of new osteons may be achieved.

The method further comprises, at step 1065, forming consecutive substantially concentric surfaces or cylinders corresponding to the lamellae within each of the circular regions. The concentric surfaces are formed until the obtained virtual model satisfies boundary conditions formed by the microstructural cortical parameters. The thickness of each of the concentric surfaces or cylinders may be constant in some examples. In other examples, the concentric surfaces may have a different thickness (a volume occupied by such a lamella may be modelled using two neighboring NURBS).

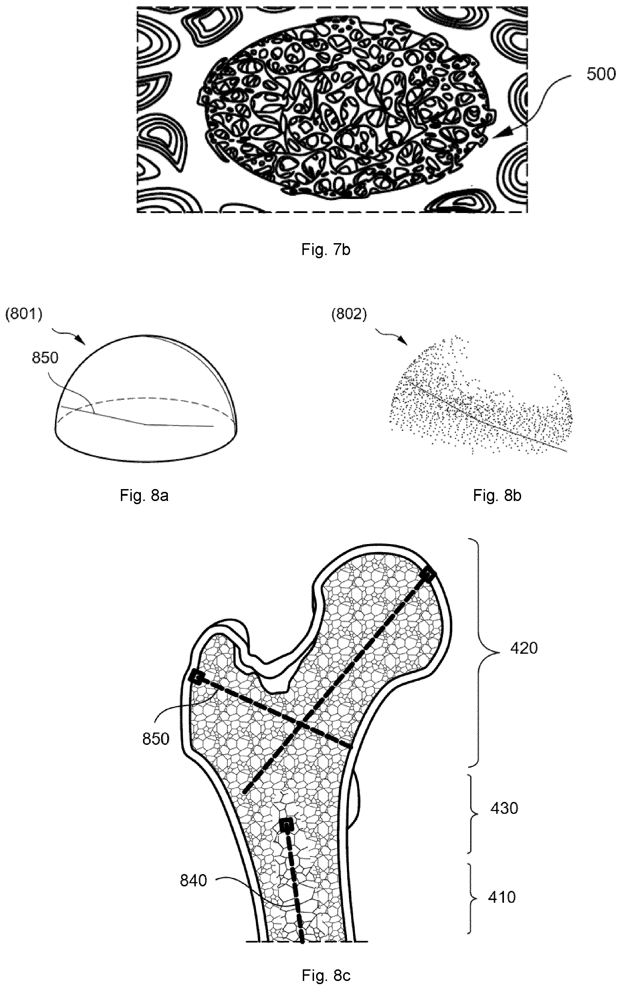

Figures 7a and 7b schematically show a perspective view of a generated virtual model of a first section 10 of bone tissue according to an example of the present disclosure. The generated virtual model is a cross section of bone tissue e.g. of a long bone of a human. The generated virtual model is a cross section of bone tissue comprising cortical bone 200 tissue and trabecular bone 300 tissue.

In particular, figure 7a shows a volume of the first section 10 of bone tissue including cortical bone tissue which has been generated using the generative design algorithm as shown in the examples of figures 6a - 6d.

As shown in figure 7a, an inside of a first concentric surface of the virtual model may be empty to resemble a Haversian canal.

In some examples, the microstructural cortical parameters may include indications of an area of the Haversian canals and/or a shape of Haversian canals, and forming a first of the consecutive substantially concentric surfaces may be based on these indications.

The generated virtual model of the example of figures 7a and 7b also comprises trabecular bone tissue which has been generated taking into account previously obtained microstructural trabecular parameters.

Figure 7b shows a closer view of the trabecular bone tissue of the virtual model. In the example, the area of the pores defined by the trabeculae varies gradually along a transverse direction of the virtual model. In addition, figure 7b also shows that the virtual model comprises a transitional region 500 between the cortical region and the trabecular region, wherein the trabecular tissue penetrates the cortical tissue.

An orientation and density of the trabecular tissue may correspond to the load axes of a real bone.

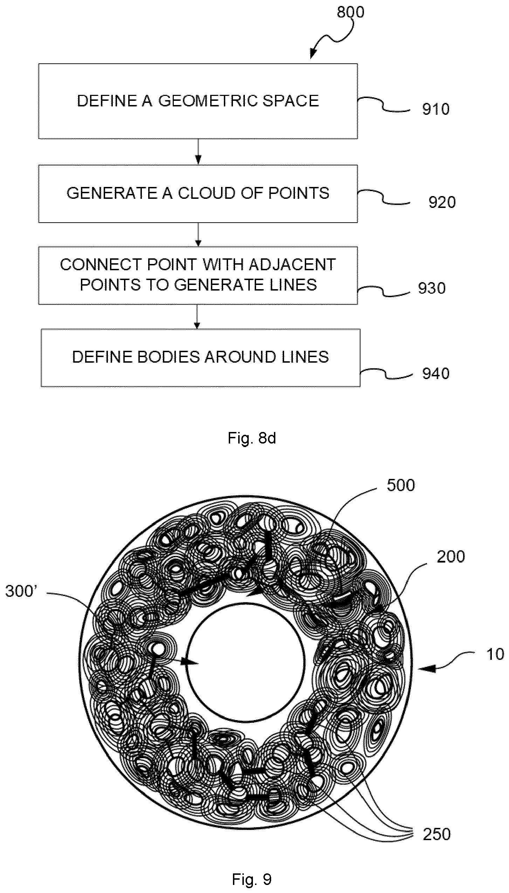

Figures 8a - 8c schematically show steps of generating a virtual model of a section of trabecular bone tissue using a generative algorithm according to an example of the present disclosure.

In some examples, generating a virtual model of a section of trabecular bone tissue may comprise, at step 910, defining a geometric space of the trabecular bone tissue and generating load axes. The geometric space of the trabecular bone tissue in particular can be defined by the inner perimeter of the cortical tissue. I.e. the inner shape of the rings of the section(s) of figure 6d can be taken as the outer perimeter of the trabecular bone tissue.

The load axes may determine the main load directions of the section of the bone. Further, the location of the load axes may be modified according to sex, age and absence or presence of pathological conditions of a subject.

In some examples, generating the load axes may comprise generating primary load axes and/or secondary load axes. In some examples, the primary load axes may be generated in the regions of the trabecular bone which may withstand higher mechanical loads e.g. the ends of the bone. The secondary load axes may be generated in the regions or sections of trabecular bone tissue which may have a lower density of the trabecular bone tissue i.e. the regions which may not withstand such high loads.

In the example of figure 8a, a hemisphere is generated to represent a head of a long bone e.g. a head of a femur, and the load axes correspond to primary load axes 850.

In other examples, other geometric space may be defined e.g. a cylinder, comprising secondary load axes.

Further, generating a virtual model of a section of trabecular bone tissue may comprise, at step 920, generating a trabeculae cloud of points, wherein a number of points may correspond to the number of trabeculae, and a density of the cloud of points may be derived from the load axes.

In some examples, a higher point density may be generated around the primary load axes and a lower point density may be generated around the secondary load axes.

The method may comprise, at step 930, connecting the plurality of trabeculae points and generating trabecular lines.

In some examples, the trabeculae points may comprise multiple sizes and shapes, which may be obtained from the plurality of microstructural parameters of trabecular bone tissue. At step 940, the trabeculae in the model may be generated by defining bodies around the trabecular lines generated in step 930. The shape and cross-section of the trabeculae may vary between different trabeculae and along individual trabeculae. As mentioned before, these indications may be derived from user input, from medical images, or from information representative for the general population or a specific population group.

Figure 8c shows a generated virtual section of trabecular tissue comprising axes of high tissue density 850 i.e. secondary load axes, and axes of low tissue density 840 i.e. primary load axes according to an example of the present disclosure.

The generated virtual model comprises a distal epiphysis 420 comprising the head of the bone generated in figures 8a and 8b, a metaphasis 430, and a portion of the diaphysis 410.

Figure 9 shows a top view of another example of a virtual model of a first cross section 10 of bone tissue including cortical bone tissue. The virtual model of the figure comprises a cortical region 200 volume and a trabecular region 300 volume. The virtual model may include the transitional region 500 between the cortical and trabecular regions.

The volume occupied by the cortical region includes cortical bone tissue. In particular, the virtual model of cortical bone tissue shown in figure 9 has a plurality of osteons 210 having different shape, size and which are formed of a different number of lamellae. In addition, the generated virtual model shown in figure 9 comprises osteons 210 having different inclination angles from each other i.e. the figure shows Haversian canals having different inclination angles. Further, the cortical bone tissue of the virtual model shown in figure 6 has been generated taking into account indications of a number of Volkmann canals 250 connecting the Haversian canals.

The virtual model of figure 9 also comprises a trabecular region 300' volume. The virtual model of the trabecular bone tissue has not been generated. The trabecular region volume may be designed to incorporate a virtual model of trabecular bone tissue. In some examples, the virtual model of trabecular bone tissue may be determined using the generative design algorithm used for determining the cortical bone tissue e.g. discussed in figures 8a - 8c. In other examples, the virtual model of figure 9 may be merged with a virtual model of trabecular bone tissue which has been obtained using a different method.

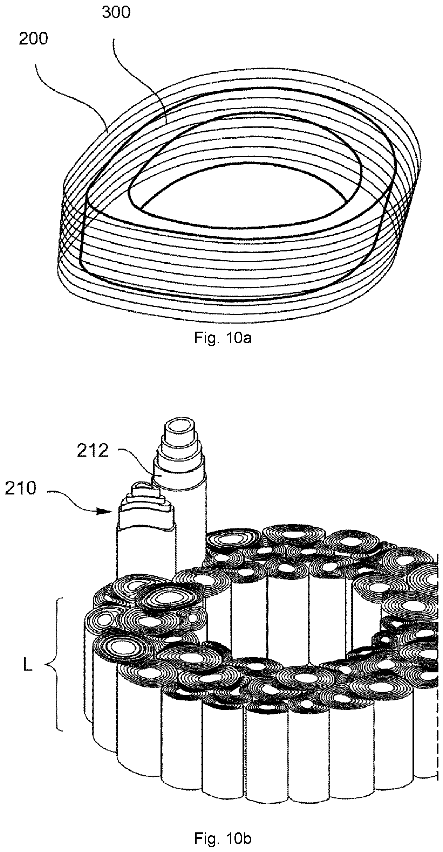

An example of another virtual model generated with the computer implemented method 100 of the present disclosure is shown in figures 10a - 10d.

Figure 10a illustrates a first step of generating a volume of the bone tissue. In the example, the generated volume of bone tissue comprises a cortical 200 region volume and a trabecular 300 region volume.

In following steps, as already addressed e.g. with reference to figures 6a - 6d, in the cortical 200 region volume a plurality of osteons is generated, each of the osteons comprising concentric surfaces corresponding to the lamellae.

Figure 10b shows multiple osteons formed by a plurality of lamellae, wherein each of the individual lamellae comprises a thickness and wherein the different lamellae have different distance between them i.e. wherein the thickness of the interlaminar space of the osteons is individualized.

In the figure, an exploded view of two of the generated osteons is shown. As may be seen, the osteons of the virtual model are formed by a plurality of lamellae separated from one another defining interlaminar spaces.

Accordingly, in the example of figure 10b, the microstructural cortical parameters used by the generative algorithm include indications of the thickness of individual lamellae forming the osteons and of the distance between the individual lamellae forming the osteons.

Further, as shown in figure 10b, a volume of the osteons within the generated cortical region may be generated by extending in a longitudinal direction the plurality of lamellae and creating cylinders.

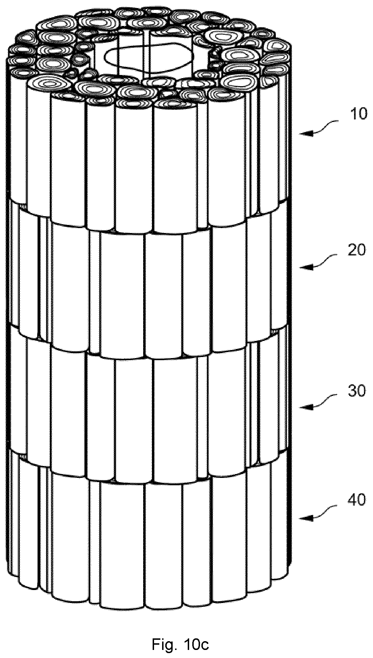

In some examples, the virtual model may include one or more additional sections, wherein the additional sections are arranged vertically below or above the first section. Figure 10c shows an example of a generated virtual model comprising four sections 10, 20, 30, 40 of cortical bone tissue according to another example of the present disclosure. The generated virtual model of figure 10c comprises the virtual model of the first section of bone tissue shown in figure 10b and may be visualized on a display.

In some examples, the osteons may have a length L corresponding to a height of the individual bone section. In other examples, osteons within a same bone section may comprise a different length L.

In figure 10c, the osteons of the different sections have a length corresponding to the height of each bone section. The model of the cortical bone may be generated by a superposition of the models of the individual sections. The model may further be adapted to generate Volkmann canals at the intersection of the individual sections to connect Haversian canals with each other if they do not line up with sections on top or below. I.e. a hollow, substantially horizontal, and a substantially cylindrical shapes is generated may be generated at the junctions between bone sections, and these connect end points of Haversian canals.

The additional sections 20, 30, 40 may be formed by the generative design algorithm, and the microstructural cortical parameters and/or the microstructural trabecular parameters may be different from the ones of the first section 10.

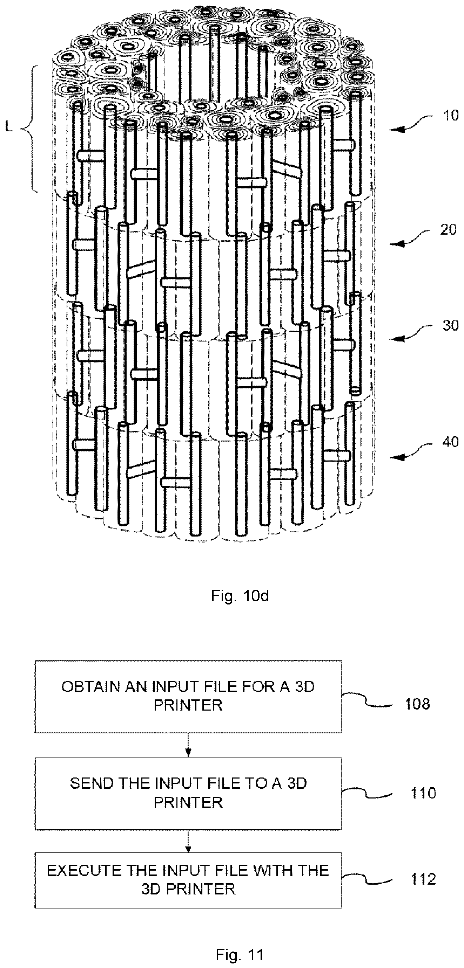

Figure 10d shows an internal view of the model of figure 10c showing that the virtual model includes Volkmann's canals connecting the plurality of osteons with adjacent osteons.

In some examples, a user may customize one or more values of the input after a section of a bone tissue has been generated e.g. a user may modify one or more values of one or more of the sections of bone tissue. As the user may give input to adapt one or more of the generated virtual sections of the bone, the resulting adapted virtual bone section may be visualized on the display e.g. in real-time.

Adapting one or more values of the micro and macrostructural parameters of the bone section may comprise the automatic adaptation of the other parameters of the virtual model of bone tissue. For example, if the number of lamellae per osteon is modified, the specific area of the osteons, the distance between the lamellae and/or the shape of the lamellae may be modified as well, and the generative deign algorithm may adapt the remaining parameters. Another example of customization of the generated virtual model of bone tissue may be the adaptation of the generated model to a different sex, age or absence or presence of pathological conditions i.e. health condition.

In addition, the computer implemented method 100 may comprise generating an input file for a 3D printer based on the obtained virtual model of bone tissue.

Once the user has generated the virtual model of the bone section according to specific needs, an input file for a 3D printer may be generated. The generated virtual model is accurate enough such that it is possible to print bone sections or entire bones imitating biological patterns.

A method for manufacturing artificial bone tissue is provided. Figure 11 shows a flow chart of the method. The method comprises, at block 108, obtaining an input file for a 3D printer, wherein the input file is based on the virtual model of the bone tissue. The method further comprises, at block 110, sending the input file to a 3D printer and, at block 112, executing the input file with the 3D printer.

In some examples, the user may enter a command for generating the input file.

In some examples, the input file for the 3D printer may be one of the following formats: .stl, .obj, .gcode, .amf, .vrml, .ply, .3mf. Any suitable format may be chosen depending on the selected printer, and depending on the applications or functionalities used to adapt the micro and macro structures of the bone section.

STL is currently the most common file format when 3D printing. STL is a standard file format that can interface between most CAD software and 3D printers. STL files can contain a triangular representation of a 3-dimensional object.

.OBJ is the second most common file format used in 3D printing. It is widely supported by 3D printers. This file is similar to .STL in that it contains 3D geometry information, such as vertex normals, geometric vertices, polygonal faces and texture coordinates.

.gcode, otherwise known as .g or .gco, is the file extension for files containing Gcode data. A .gcode file may be created by a slicing program, which turns a CAD drawing into a string of code that a suitable 3D printer can understand. These are just some of the more common input file formats for 3D printers, however it should be clear that many further alternatives are available.

The method may further comprise printing the generated section of the long bone with additive manufacturing. The 3D printer may be configured to execute the input file with stereolithography or SLA.

In some examples, a combination of resin and hydroxyapatite may be used to print the long bone section. A customized synthetic bone may be printed via additive manufacturing, which imitates behavior patterns of biological bones. The bones may be used as medical or educational models.

In some examples, printing the one or more generated virtual sections of the long bone may comprise printing the sections at different scales.

A section or an entire long bone may be printed at a real scale i.e. at a scale 1.1. A synthetic bone may be obtained comprising a microarchitecture and a microarchitecture which mimic biological micro and macro architectures of biological bone tissue. In particular, the synthetic bone may comprise a cortical region made of osteon-based structures and a trabecular region made of trabeculae-based structures, imitating the spatial distribution of biological bone mass. The synthetic bone may reproduce crack propagation through the various bone microstructures. In other examples, a long bone may be printed at a higher scale e.g. 3:1.

In some examples, a synthetic bone in its entirety may be printed using additive manufacturing. The synthetic bone may be printed from an input file generated by any of the methods disclosed herein.

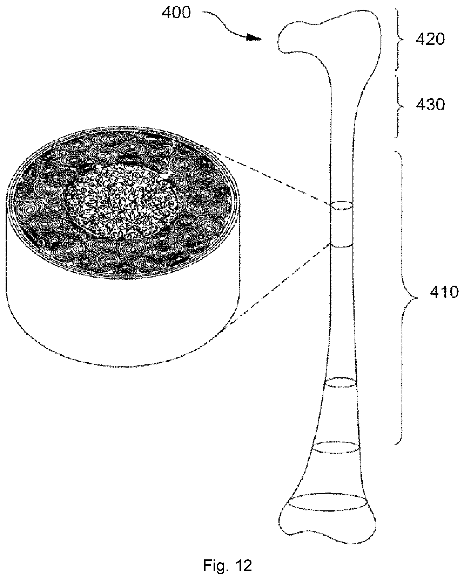

Figure 12 schematically shows an example of a synthetic long bone 400 manufactured by additive manufacturing. The bone has been printed from a virtual model of a long bone which has been generated according to the computer implemented method 100.

The printed long bone 400 comprises the diaphysis 410, the central section of the bone, proximal and distal epiphysis 420, the ends of the long bone, and metaphasis 430, located between the diaphysis 410 and the epiphysis 420.

The long bone 400 schematically shown in figure 12 comprises a plurality of sections of long bone, arranged vertically below or above of each other. Each section comprises micro and macro architectures which may depend on the longitudinal position of the section within the bone i.e. the long bone may not have the same micro architecture in the central region of the diaphysis than at a region of the metaphasis.

The synthetic long bone 400 comprises a cortical region comprising a plurality of osteon-based structures and a trabecular region comprising a plurality of trabeculae-based structures defining a number of pores. Each of the osteon-based structures is defined at least by a plurality of concentric lamellae-based rings. In addition, the number of the pores of the trabecular region varies gradually along a transversal direction of the bone.

Experiments carried out with artificial bones and sections of bones obtained by methods according to the present disclosure have shown that the propagation of cracks in artificial bone very closely mimics the propagation of cracks in natural bone. Without wishing to be bound by theory, it is believed that the identification of osteons as being built up of individual lamellae, and the ability to mimic the variation of porosity in trabecular bone are two key factors that improve the ability to mimic natural bone with respect to prior art proposals.

In a further aspect of the present disclosure, a computer program comprising instructions which when the program is executed by a computer, cause the computer to carry out the computer implemented method 100 is provided.

A computer-readable data carrier may have stored thereon the computer program comprising the instructions which when executed cause the computer to carry out the method 100.

In yet a further aspect, a data processing apparatus is provided which comprises a processor that is configured to perform the method 100.

The various illustrative logical blocks, modules, and circuits described in connection with the disclosure herein may be implemented or performed with one or more general-purpose processors, a digital signal processor (DSP), cloud computing architecture, an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The computer program may be in the form of source code, object code, a code intermediate source and object code such as in partially compiled form, or in any other form suitable for use in the implementation of the processes. The carrier may be any entity or device capable of carrying the computer program.

If implemented in software/firmware, the functions may be stored on or transmitted over as one or more instructions or code on a computer-readable medium. Computer-readable media includes both computer storage media and communication media including any medium that facilitates transfer of a computer program from one place to another.

A storage media may be any available media that can be accessed by a general purpose or special purpose computer. By way of example, and not limitation, such computer-readable media can comprise RAM, ROM, EEPROM, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor.

Also, any connection is properly termed a computer-readable medium. For example, if the software/firmware is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, includes compact disc (CD), laser disc, optical disc, digital versatile disc (DVD), floppy disk and Blu-ray disc where disks usually reproduce data magnetically, while discs reproduce data optically with lasers. Combinations of the above should also be included within the scope of computer-readable media.

For completeness, a number of aspects of the present disclosure are set out in the following numbered clauses:
Clause 1. A computer-implemented method for generating a virtual model of a first section of bone tissue including cortical bone tissue, the method comprising:
   obtaining a plurality of macrostructural parameters of the bone tissue;
   obtaining microstructural cortical parameters of the cortical bone tissue, wherein the microstructural cortical parameters include:
      - an indication of a number of osteons,
      - an indication of a cross-sectional area of the osteons
      - a number of lamellae forming the osteons,
      - an indication of locations of osteons in the section of bone tissue, and
      - an indication of dominance of the osteons
   generating the virtual model of the first section of bone tissue using a generative design algorithm, wherein the generative design algorithm comprises:
      defining a geometric space of the section of bone tissue, wherein an outer geometric shape of the virtual model is substantially in accordance with the macrostructural parameters;
      generating a cortical cloud of points, wherein a number of points of the cortical cloud of points corresponds to the number of osteons, and a location of the points is derived from the indication of the locations of the osteons;
      segmenting the geometric space into a plurality of substantially circular regions, wherein each of the regions comprises one of the points;
      defining an outer surface of the osteons based on the substantially circular regions, wherein at an intersection between two adjacent circular regions, the outer surface of the osteons is determined by the substantially circular regions and the level of dominance of the osteons such that a portion of the outer perimeter of a more dominant osteon is defined by the substantially circular region and a portion of the outer perimeter of a less dominant osteon is defined by the substantially circular region of the adjacent osteon; and
      forming consecutive substantially concentric surfaces corresponding to the lamellae within each of the circular regions,
      wherein the forming of concentric surfaces continues until the obtained virtual model satisfies boundary conditions formed by the microstructural cortical parameters.
Clause 2. The method of clause 1, wherein an inside of an innermost concentric surface of the virtual model is empty to resemble a Haversian canal.
Clause 3. The method of clause 1 or 2, wherein the microstructural cortical parameters further include indications of a thickness of individual lamellae forming the osteons and/or a distance between the individual lamellae forming the osteons.
Clause 4. The method of any of clauses 1 - 3, wherein the microstructural cortical parameters further include indications of an area of the Haversian canals and/or a shape of Haversian canals, wherein forming an innermost of the consecutive substantially concentric surfaces is based on these indications.
Clause 5. The method of any of clauses 1 - 4, wherein the microstructural cortical parameters further include indications of a number of Volkmann canals connecting the Haversian canals.
Clause 6. The method of any of clauses 1 - 5, wherein the osteons have a length corresponding to a height of the section.
Clause 7. The method of any of clauses 1-6, wherein the section of bone tissue further comprises trabecular bone tissue, and wherein the method further comprises:
   obtaining a plurality of microstructural trabecular parameters of the trabecular bone tissue; and
   determining the virtual model of the section of bone tissue comprises generating a virtual model of the trabecular bone tissue.
Clause 8. The method of clause 7, wherein the microstructural trabecular parameters include indication of a number of trabeculae, an indication of a distribution of the trabeculae, an indication of an area and/or cross-section of the trabeculae.
Clause 9. The method of clause 7 or 8, wherein the virtual model of trabecular bone tissue is determined using the generative design algorithm, and wherein the generative design algorithm further comprises:
   defining a geometric space of the trabecular bone tissue;
   generating a trabecular cloud of points, wherein a number of points of the trabecular cloud of points is derived from the number of trabeculae;
   connecting the points of the trabecular cloud of points with each of the adjacent point of the trabecular cloud of points to define trabecular lines;
   generating the trabeculae by defining bodies around the trabecular lines in accordance with the indication of the area and/or cross-section of the trabeculae.
Clause 10. The method of any of clauses 7-9, comprising obtaining one or more axes of increased density or decreased density, and wherein generating the trabecular cloud of points takes into account the axes of increased or decreased density.
Clause 11. The method of any of clauses 7 - 10, wherein the geometric space of the trabecular bone tissue is defined by the outer geometric shape of the virtual model of the cortical tissue.
Clause 12. The method of any of clauses 1 - 11, wherein the virtual model of bone tissue includes one or more additional sections, wherein the additional sections are arranged vertically below or above the first section.
Clause 13. The method of clause 12, wherein the additional sections are also formed by the generative design algorithm, and wherein the microstructural cortical parameters and/or the microstructural trabecular parameters are different from the first section.
Clause 14. The method of any of clauses 1 - 13, further comprising:
   generating an input file for a 3D printer based on the obtained virtual model.
Clause 15. The method of clause 14, wherein the input file for the 3D printer is in one of the following formats: .stl, .obj, .gcode, .amf, .vrml, .ply, .fbx, .3mf.
Clause 16. The method of any of clauses 1 - 15, wherein the plurality of macrostructural parameters and/or the plurality of microstructural cortical parameters and/or the plurality of microstructural trabecular parameters are obtained from user input.
Clause 17. The method of any of clauses 1 - 15, wherein the plurality of macrostructural parameters and/or the plurality of microstructural cortical parameters and/or the plurality of microstructural trabecular parameters are derived from medical images.
Clause 18. The method of any of clauses 1 - 17, wherein the plurality of macrostructural parameters and/or the plurality of microstructural cortical parameters and/or the plurality of microstructural trabecular parameters are average values for subjects belonging to a population, and wherein
   the population of subjects is based on sex, age and absence or presence of pathological conditions of a subject.
Clause 19. The method of clause 18, wherein the pathological conditions include comprise one of osteoporosis or arthrosis.
Clause 20. A computer program comprising instructions which, when the program is executed by a computer, cause the computer to carry out the method of any of clauses 1 - 19.
Clause 21. A computer-readable data carrier having stored thereon the computer program of clause 20.
Clause 22. A data carrier signal carrying the computer program of clause 20.
Clause 23. A data processing apparatus comprising a processor configured to perform the method of any of clauses 1 - 19.
Clause 24. A method for manufacturing artificial bone tissue, comprising:
   obtaining an input file for a 3D printer in accordance with clause 15;
   sending the input file to a 3D printer; and
   executing the input file with the 3D printer.
Clause 25. A method of clause 24, wherein the 3D printer is configured to execute the input file with stereolithography.
Clause 26. A method of clause 25, wherein the 3D printer uses hydroxyapatite.
Clause 27. Artificial bone tissue as obtained by the method of any of clauses 24 - 26.

This written description uses examples to disclose the teaching, including the preferred embodiments, and also to enable any person skilled in the art to practice the teaching, including making and using any devices or systems and performing any incorporated methods. The patentable scope is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims. Aspects from the various embodiments described, as well as other known equivalents for each such aspects, can be mixed and matched by one of ordinary skill in the art to construct additional embodiments and techniques in accordance with principles of this application. If reference signs related to drawings are placed in parentheses in a claim, they are solely for attempting to increase the intelligibility of the claim, and shall not be construed as limiting the scope of the claim.

## Claims

1. A computer-implemented method for generating a virtual model of a first section of bone tissue including cortical bone tissue, the method comprising:
obtaining a plurality of macrostructural parameters of the bone tissue;
obtaining microstructural cortical parameters of the cortical bone tissue, wherein the microstructural cortical parameters include:
- an indication of a number of osteons,
- an indication of a cross-sectional area of the osteons
- a number of lamellae forming the osteons,
- an indication of locations of osteons in the section of bone tissue, and
- an indication of dominance of the osteons
generating the virtual model of the first section of bone tissue using a generative design algorithm, wherein the generative design algorithm comprises:
defining a geometric space of the section of bone tissue, wherein an outer geometric shape of the virtual model is substantially in accordance with the macrostructural parameters;
generating a cortical cloud of points, wherein a number of points of the cortical cloud of points corresponds to the number of osteons, and a location of the points is derived from the indication of locations of the osteons;
segmenting the geometric space into a plurality of substantially circular regions, wherein each of the regions comprises one of the points;
defining an outer surface of the osteons based on the substantially circular regions, wherein at an intersection between two adjacent circular regions, the outer surface of the osteons is determined by the substantially circular regions and the level of dominance of the osteons such that a portion of the outer perimeter of a more dominant osteon is defined by the substantially circular region and a portion of the outer perimeter of a less dominant osteon is defined by the substantially circular region of the adjacent osteon; and
forming consecutive substantially concentric surfaces corresponding to the lamellae within each of the circular regions;
wherein the forming of concentric surfaces continues until
the obtained virtual model satisfies boundary conditions formed by the microstructural cortical parameters.

2. The method of claim 1, wherein the microstructural cortical parameters further include indications of a thickness of individual lamellae forming the osteons and/or a distance between the individual lamellae forming the osteons.

3. The method of claim 1 or 2, wherein the microstructural cortical parameters further include indications of a number of Volkmann canals connecting the Haversian canals.

4. The method of any of claims 1-3, wherein the osteons have a length corresponding to a height of the section.

5. The method of any of claims 1-4, wherein the section of bone tissue further comprises trabecular bone tissue, and wherein the method further comprises:
obtaining a plurality of microstructural trabecular parameters of the trabecular bone tissue;
and determining the virtual model of the section of bone tissue comprises generating a virtual model of the trabecular bone tissue.

6. The method of claim 5, wherein the microstructural trabecular parameters include indication of a number of trabeculae, an indication of a distribution of the trabeculae, an indication of an area and/or cross-section of the trabeculae.

7. The method of claim 5 or 6, wherein the virtual model of trabecular bone tissue is determined using the generative design algorithm, and wherein the generative design algorithm further comprises:
defining a geometric space of the trabecular bone tissue;
generating a trabecular cloud of points, wherein a number of points of the trabecular cloud of points is derived from the number of trabeculae;
connecting the points of the trabecular cloud of points with each of the adjacent point of the trabecular cloud of points to define trabecular lines;
generating the trabeculae by defining bodies around the trabecular lines in accordance with the indication of the area and/or cross-section of the trabeculae.

8. The method of any of claims 5 - 7, comprising obtaining one or more axes of increased density or decreased density, and wherein generating the trabecular cloud of points takes into account the axes of increased or decreased density.

9. The method of any of claims 5 - 8, wherein the geometric space of the trabecular bone tissue is defined by the outer geometric shape of the virtual model of the cortical tissue.

10. The method of any of claims 1 - 9, wherein the virtual model of bone tissue includes one or more additional sections, wherein the additional sections are arranged vertically below or above the first section.

11. The method of any of claims 1 - 10, further comprising:
generating an input file for a 3D printer based on the obtained virtual model.

12. A method for manufacturing artificial bone tissue, comprising:
obtaining an input file for a 3D printer in accordance with claim 11;
sending the input file to a 3D printer; and
executing the input file with the 3D printer.

13. The method of claim 12, wherein the 3D printer is configured to execute the input file with stereolithography, in particular wherein the 3D printer uses hydroxyapatite.

14. The method of any of claims 1 -13, wherein the plurality of macrostructural parameters and/or the plurality of microstructural cortical parameters and/or the plurality of microstructural trabecular parameters are obtained from user input, or are derived from medical images, or correspond to average values for subjects belonging to a population, wherein the population of subjects is based on sex, age and absence or presence of pathological conditions of a subject.

15. Artificial bone tissue obtained by the method of claim 12 or 13.
